Europäisches Patentamt

European Patent Office

Office européen des brevets

(11) Publication number : **0 512 857 A2**

# EUROPEAN PATENT APPLICATION

(21) Application number : 92304167.7

(22) Date of filing : 08.05.92

(51) Int. Cl.⁵ : **A61K 35/74, A23L 1/48**

(30) Priority : **10.05.91 JP 135532/91**

(43) Date of publication of application :
**11.11.92 Bulletin 92/46**

(84) Designated Contracting States :
**DE ES FR GB IT**

(71) Applicant : **Yabiki, Terutake**
**26-16 Miyamae 2-chome**
**Sakura-shi, Chiba (JP)**

(71) Applicant : **Yabiki, Setsuko**
**26-16 Miyamae 2-chome**
**Sakura-shi, Chiba (JP)**

(72) Inventor : **Yabiki, Terutake**
**26-16 Miyamae 2-chome**
**Sakura-shi, Chiba (JP)**
Inventor : **Yabiki, Setsuko**
**26-16 Miyamae 2-chome**
**Sakura-shi, Chiba (JP)**

(74) Representative : **Holmes, Michael John et al**
**Frank B. Dehn & Co. Imperial House 15-19**
**Kingsway**
**London WC2B 6UZ (GB)**

(54) Composition comprising bacterial culture residue and decapsulated bacterial cells and its use.

(57) Non-toxic antidisease preparations and food additives which prevent infective microorganism disease, especially in humans, and in particular diseases caused by herpes viruses are disclosed.

The antidisease preparations and food additives contain a mixture of optionally dehydrated bacterial culture residue concentrate and decapsulated bacterial cells as the active ingredient.

EP 0 512 857 A2

The present invention relates to non-toxic antiherpesvirus and antidisease preparations and food additives which improve the clinical symptoms of the disease arising from microbes such as bacteria or viruses including a herpesvirus, and present preventive effects thereof for,humans, in particular, for babies, infants, or adults.

In the human being, particularly in infancy and senescence, there is a high incidence of infectious diseases in the digestive organs or the respiratory system caused by bacteria, viruses, or the like as due to reduced functioning of immunocytes and the immune system.

Although a variety of antibacterial agents such as antibiotics and sulphur drugs are currently used for the prevention and treatment of these diseases, the effects are not necessarily sufficient. Moreover, there is a recent tendency towards the restriction of antibacterial agents such as antibiotics due to the appearance of drug resistant bacteria and side effects thereof.

Therefore, it is highly desirable to develop drugs having preventive and treating effects for these infectious diseases, other than antibacterial agents such as antibiotics.

In particular, many human beings are infected with herpes simplex virus during the ages of 2 to 5, though only some of them are recognized to be clinically diseased. Nevertheless, troublesome diseases which occur repetitively or relapse frequently are herpesvirus hominis infectious diseases such as human herpes labialis, herpes genitalis, herpetic keratitis, and the like caused by the herpes simplex virus. Further, the herpes simplex virus may also cause infant acute ,herpetic gingivostomatitis, Kaposi's disease, herpes dermatitis, traumatic herpes, herpetic meningoencephalitis, and newborn generalized helpetic infectious disease.

Vaccines, antibiotics, synthetic antibacterial substances, or the like effective in the prevention and treatment for herpesvirus hominis infectious diseases have not yet been developed, so the present condition is such that the appearances of these diseases must be solely treated with symptomatic therapy.

Also, Aujeszky's disease, which is otherwise referred to as pseudorabies, is an acute infectious disease caused by herpesvirus suis.

Also affected by such disease are animals such as hogs, cattle, horses, goats, rabbits, dogs, cats, mink, foxes, rats, and the like, which presents a localized severe pruritus in animals other than hogs. When the condition of the disease is advanced, paralysis of medulla oblongata, fauces, jaw, and the like, sialorrhea, dyspnea, and irregular heart-beat may result in death.

For hogs which are at least 30-70 days old, the severity of the disease may often be only mild in spite of their higher sensitivity to such disease. However, recently in areas highly affected by such disease, there are many cases where,in hogs weighing 30Kg to 80Kg, that disease develops into a respiratory disease such as pneumonia, thereby leading to death. In general, the condition of a young pig tends to be graver, resulting in a high mortality rate.

When the pregnant sows are affected by such disease, they have often miscarry. Prevention and treatment for such disease, include a variety of procedures such as prompt isolation or artificial selection of animals diseased or affected by such disease; prevention of cramped living conditions; disinfection to prevent the spread of the disease through breeders, breeding grounds, tools and feeds; and a high dosage of antibiotics, synthetic antibacterial substances, or nutrients such as vitamins, minerals, and the like for the prevention of respiratory disease complications such as pneumonia, but none of these precautions are fully effective. Further administration of an attenuated vaccine or an inactive vaccine has been tried, which succeeds in suppressing the crises, but fails to block or prevent the infection or the carrier of virulent field viruses.

In this manner, human beings and domestic animals (including horses) suffer badly from herpesvirus, the prevention and treatment of which are difficult.

In this context, there is a trend toward large dosages of medicaments in order to prevent and treat various diseases(including herpes virus infectious diseases such as human herpes labialis or Aujeszky's disease), which results in adverse side effects from medicaments, or residual medicaments in the livestock products and seafoods. Thus, a prevention against and treatment for the diseases is strongly desired, especially one which shows no side effects caused by the medicaments and so is safer as well as labor saving

Recently it has been found that an ingredient of the cell wall by microbes such as BCG (Bacillus Calmette-Guerin) or Nocardi and which is thought to be apeptidoglycan has an immunity improving function ("Journal of National Cancer Institute" vol.52, P.1571, 1974; "Gann"; vol. 69, p.669, 1976; "Cancer Immunology and Immunotherapy" vol. 4, p. 95, 1978).

Moreover, an antineoplastic drug whose active ingredient is an enzyme-treated cell wall of a bacterium of Bifidobacterium (Japanese Patent Publication No. 1987-36006), and a drug to prevent and treat diarrhea of domestic animals and domestic fowl whose active ingredient is a physically crushed and enzyme degraded cell wall of that bacterium (Japanese Patent Publication No. 1987-34732) are also known.

Thereafter, attention was drawn to gram-positive bacteria which are widely distributed throughout the natural world and free from the risk of including endotoxin. It has been found that the peptidoglycan extracted from a part of the gram-positive bacteria cause an immunity improvement effect to the pig and also a weight gain effect ("Japanese Journal of Veterinary Science", vol.49, p.235, 1987).

Furthermore, an immunity improvement agent for domestic animals whose active ingredient is bacteria with their capsules removed is also disclosed (Japanese Patent Laid-open No. 1990-11519).

Moreover, a diarrhea prevention agent which can be added to the feed of domestic animals, the active ingredient being a lactobacillus culture residue obtained by substantially removing the bacteria from ordinary lactobacillus culture solution is also known (Japanese Patent Laid-open No. 1987-104552).

Also disclosed is a diarrhea prevention agent for domestic animals to be added to food, which is a mixture of carboxylic acid(s) and a substance obtained through the concentration or dehydration of a culture solution for culturing lactobacillus or centrifugal supernatent of the culture solution (Japanese Patent Laid-open Publication No. 1989-98446).

As described above an additive is known which prevents the diarrhea of domestic animals, by the utilization of the immunity improvement effect of bacterial cells obtained by removing the capsules from the gram-positive bacteria which are wide spread in nature. Nevertheless, the virulence of the agent is a problem in case it is applied to human beings, so the target pathogenic microorganisms are limited to Escherichia coli and the like.

It is therefore an object of the present invention to provide an anti-herpesvirus for which the target pathogen range covers a variety of microorganisms including herpesvirus and which is suitable for human beings without any anxiety as to its innocuousness, and also to provide preparations and food additives which increase immunity and prevent infection of digestive organs, respiratory system, or other parts of the body or improve clinical conditions.

Through a study to develop food additives which contribute to an improvement in human health, the present inventors have found that a mixture of concentrated or dehydrated bacterial culture residue and bacterial cells without capsules results in a higher defensive activity against human microbes which have an enhanced pathogenicity such as colibacillus, Salmonella Enteritidis, Salmonella Typhimurium, and the like as compared with the case where they are separately used, and also a markedly effective defensive activity against viruses such as herpesvirus. Further, the mixture of the bacterial cells without capsules and the concentrated or dehydrated bacterial culture residue is not itself virulent, so that there is nothing of concern regarding the safety of the additive to human foods.

The present invention is directed to an antiherpesvirus product whose active ingredient is a mixture of condensed or dehydrated bacterial culture residues and bacterial cells with capsules removed, and to antidisease preparations and food additives for enhancing immunity.

Microorganisms used in this invention may be any bacteria or yeasts, preferably genus Bifidobacterium such as Bifidobacterium infantis, non-pathogenic genus Bacillus such as Bacillus subtilis, genus Lactobacillus such as Lactobacillus acidophilus, and non-pathogenic strains of genus Streptococcus such as Streptococcus faecalis, and yeasts such as Kluyversmyces lactis and Saccharomyces cerevisiae. Especially preferred are Bifidobacterium bacteria.

Bacterial cells without capsules used in the present invention can be obtained by treating the bacterial cells enzymatically or with a surfactant, or stirring or shaking them. In each case the bacterial cell wall should remain intact after decapsulation. In the case of surfactant treatment, the bacterial cells may be suspended into the surfactant solution, or they may be suspended in water or a buffer solution prior to the addition of surfactant. Before these procedures, it is desirable that autolysins be inactivated to protect the cell walls of the microorganism.

The thus obtained decapsulated bacterial cells are bacterial cells whose capsules have been removed to expose the cell wall. In gram-positive bacteria, for example, the cell wall is of a baggy structure in which peptidoglycan is covalently bonded. The structure particular to a bacterium includes peptidoglycan and polysaccharide or teichoic acid as its principal ingredients. The cell wall in outward contact with the cytoplasmic membrane serves to hold the structure of the bacterial cell and to protect the cytoplasmic membrane and its functions from various outside stimuli. In particular, the peptidoglycan portion is not only indispensable for enhancing immunity, but also contributes largely to the maintenance of the entire structure of the bacterial cell. In other words, the peptidoglycan, which is wholly non-water-soluble and a physically rigid giant polymer, has a three-dimensional structure in which glucan chains comprising N-acetyl-glucosamine and N-acetylmuramic acid are combined with peptido chains to form a mesh.

In order to obtain a condensed or dehydrated substance of the bacterial culture residue used in the present invention, microorganisms are inoculated into a synthetic medium or semi-synthetic medium, cultured in accordance with usual method, gathered by means of centrifugal separation or dialysis, and cleared from the culture solution to obtain a culture residue. Then, the culture residue is further condensed by 5 to 20 times into a concentrate having a solid content of 40 to 70%. The dehydrated substance can be obtained by means of spray drying or freeze drying of such concentrate or otherwise. The term "culture residue" refers to both the concentrate and dehydrate as described above.

The culture residue thus obtained contains a lot of useful culture metabolites such as amino acids, vitamins and the like which cultured microorganisms yield, is rich in nutrition, and has extremely superior physiological and immunological properties. Also, from the viewpoint of effective utilization of by-products produced in the mass culture of microorganisms, the present invention is of economical and environmental significance.

The present invention comprises the thus obtained concentrate or dehydrated product of bacterial culture residue, and bacterial cells whose capsules are removed. In this case, it is preferable that a mixing ratio based on dry weight between the bacterial cells without capsules and the bacterial culture residue lie within the range of 0.0002 : 1 to 0.5 : 1. The resultant products may be powdered or fined into-particles with the aid of an edible weighting agent such as starch, dextrin, powdered skim milk and the like, or dissolved into water to thereby obtain an antimicroorganism and antidisease food additive.

These antiherpesvirus and antidisease preparations and food additives may either be taken orally as they enhance resistance to herpesvirus and disease in babies, infants or adults, or may be added to foods, for example, health drinks, soft drinks, cakes, baby drinks, baby cakes, and baby foods.

The antiherpesvirus and antidisease preparations in accordance with the present invention comprise a concentrate of a bacterial culture residues and bacterial cells whose capsules have been removed, both being obtained by the above method. In this case, it is preferred that a compositional ratio based on dry weight between the bacterial cells without capsules and the bacterial culture residue be within a ratio of 0.001:1 to 0.5:1. A mixture having the above compositional ratio is added to an edible weighting agent such as lactose and soluble starch within the range of 0.1 to 10wt%, to obtain any type of powders, granules, tablets, or solutions. For tablets, a small amount (0.5wt% or less) of talc or titanium oxide is preferably added to the mixture together with CMC (carboxymethyl cellulose), 0.1wt% or less serving as a paste; and the like. The types of medication described above can be appropriately selected according to age or physical condition of the user.

When the antiherpesvirus and antidisease preparations are taken orally, one dose of 0.01g to 3g should be taken once to several times a day.

Also, when the mixed food additive is added to food, an additive content in the food lies within the range of 0.01 to 5.0wt%, preferably 0.05 to 1.0wt%.

Examples of 'the present invention and test results of immunity enhancement effects and virulence of the antivirus and antidisease preparations in accordance with the present invention will be hereinafter described. It should be noted that the present invention is not limited to the following examples.

EXAMPLE 1

[a] Production of Bacterial Culture Residue

The strains of <u>Bifidobacterium</u> <u>infantis</u> [ATCC (American Type Culture Collection) 15697] were inoculated on a liquid culture medium 100 l (pH 6.8) consisting of enzyme-decomposed casein 2.0%, peptone 1.0%, yeast extract 1.0%, lactose 2.0%, fracto-oligosaccharide 0.41%, cysteine 0.02% (percentage by weight), cultivated for 18 hours at the temperature of 37°C, and then centrifuged for 15 minutes at 8000 rpm by means of a refrigerated centrifuge, to yield about 980g of wet bacterial cells. Also, about 95 l of culture residue cleared of the bacterial cells was obtained.

[b] Process of Producing Bacterial Cells without Capsules

500g of wet bacterial cells out of strains of <u>Bifidobacterium</u> <u>infantis</u> ATCC 15697 obtained in accordance with Example 1 [a] were suspended in 1.5l 0.05M phosphate buffer (pH 7.0) , heated for 45 minutes at 70°C to inactivate autolytic enzymes possessed by the bacteria, abruptly cooled in running water, and stirred up for 24 hours at 37°C. Thereafter, bacterial cells were harvested through 3500 rpm centrifugation for 20 minutes, suspended once in purified water, and subjected to centrifugal cleaning for 15 minutes at 8500 rpm to harvest the bacterial cells. Then, the bacterial cells were lyophilized to obtain 65g of <u>Bifidobacterium infantis</u> cells whose capsules have been eliminated in the form of dehydrated substances.

In thus obtained bacterial cells, hexosamine (containing N-acetylglucosamine and N-acetylmuramic acid as its main constituent) which is an immunity enhancement substance was present in the amount 2.89% or 3.98% based on the analyses of the Morgan-Elson and Dische-Bordenfreund method (indole / hydrochloric acid method) respectively.

[c] Production of Dehydrated Bacterial Culture Residue

90 l of culture residue cleared of bacterial cells from strains of <u>Bifidobacterium</u> <u>infantis</u> ATCC 15697 ob-

tained in accordance with Example 1 [a] was taken out and lyophilized by means of suitable apparatus, to obtain about 4.8 Kg of culture residue powder for strains of Bifidobacterium infantis ATCC 15697.

[d] Production of Food Additive

1g of bacterial cells without capsules obtained in Example 1 [b] and 50g of culture residue powder cleared of bacterial cells obtained in Example 1 [c] were mixed with 849g of powdered skim milk and 10g of sugar to obtain food additives.

EXAMPLE 2

The product of Example 1 [b] and 50g of culture residue powder free from bacterial cells obtained in Example 1 [c] were added to 800g of soluble starch and 10g of lactose and a small amount of talc and CMC, to obtain an antiherpesvirus granule.

TEST EXAMPLE 1

Test for Human Herpes Labialis Virus

Using the bacterial cell powder and culture residue powder produced in Example 1 [b] and Example 1 [c] respectively, the preparations A (0.05g of bacterial cells powder), B (0.25g of culture residue powder), and C (0.05g of bacterial cells powder + 0.25g of culture residue powder) were prepared individually.

To three groups of three patients (male 1, female 2) of 26 to 49 years old who suffered a relapse of human herpes labialis (Pathogenic organism: Herpesvirus hominis) between the last one to three months, preparations A, B, or C previously prepared were administered twice a day (morning and evening) every day for 12 months, to examine relapse conditions of the disease. The groups are lavelled A, B and C according to the particular preparation they received.

Table 1 shows the results.

[TABLE 1]

| Test Group | Number of Patients | Relapse None | Slight Relapse Once | Slight Relapse Several Times | Unchanged from before Dosage Relapse Observed |
|---|---|---|---|---|---|
| A(Comparative Group) | 3 | 2 | – | 1 | – |
| B(Comparative Group) | 3 | – | 1 | 1 | 1 |
| C(Preparation Group of Invention) | 3 | 3 | – | – | – |

As can be clearly seen from the above test results, in Group A (comparative group) one patient suffered slight relapses several times, and the other two had no relapses. In Group B (comparative group) the number of patients who suffered a slight relapse once, patients who suffered slight relapses several times, and patients who had relapse unchanged from before the dosage were one respectively. On the other hand, in Group C (test group) none of the three patients had a relapse. From these facts, it has been recognized that the mixture of the bacterial cells powder and the culture residue powder presents a higher relapse prevention effects against human herpes labialis compared with single dosage in Groups A and B.

TEST EXAMPLE 2

Test for Hog herpesvirus

[a]

A (0.02 wt% of bacterial cell powder), B (0.5 wt% of culture residue powder) and C (0.01 wt% of bacterial cell powder + 0.5 wt% of culture residue powder) were added to feed for raising hogs for market to produce a test feed. As the bacterial cell powder and culture residue powder, the products in Example 1 [b] and Example 1 [c] were used respectively.

Previously prepared feeds containing A, B, or C as additives and additive-free feed were each fed to one group of 20 pigs of 50 to 60 days after birth. The pigs were infected with hog herpesvirus type I (Aujeszky's disease virus) until they were transferred to a slaughterhouse so that accident ratios such as their deaths and the like could be compared.

The test results are shown in Table 2.

6

[TABLE 2]

| Test Groups | Number of Specimens | Accident Ratio (%) |
|---|---|---|
| Control Group | 20 | 30.0 * (6/20) |
| A Additive Group (Comparative Group) | 20 | 5.0 (1/20) |
| B Additive Group (Comparative Group) | 20 | 15.0 (3/20) |
| C Additive Group (Group to which preparations of the Invention is added) | 20 | 0.0 (0/20) |

An asterisk (*) in the table designates death due to the complication of pneumonia.

As is apparent from the above test results, in the additive-free control group the accident ratio was 30.0% which is rather high, while in Group with B added (Comparative Group) and in the Group with A added (Comparative Group) they were 15.0% and 5.0 % respectively which is considered to be relatively low. These pigs died due to the complication of pneumonia. On the contrary, in the Group with C (Group which received preparations of the present invention) the ratio was 0.0%. Consequently, it was verified that the mixture of the bacterial cell powder and the culture residue powder has a higher anti-disease effect compared with a single addition of A or B.

[b]

A (0.05 wt% of bacterial cell powder), B (0.5 wt% of culture residue powder), and C (0.01 wt% of bacterial cell powder + 0.5 wt% of culture residue powder) were added to a feed for raising hogs for market, to prepare test feeds. As the bacterial cell powder and the culture residue powder the products in Example 1 [b] and [c], respectively were used.

A feed containing any one of A, B, or C mentioned above as an additive and an additive-free feed were each fed to a group consisting of ten breeding sows infected with hog herpesvirus type I (Aujeszky's disease virus) from about two weeks before mating through parturition until weaning, to compare the number of death abortions, the number of born baby pigs, and the number of pigs at the time of weaning.

Test results are shown in Table 3.

[TABLE 3]

| Test Groups | Number of Specimens | Number of Death Abortions | Number of Born pigs | Number of Pigs at Weaning* |
|---|---|---|---|---|
| No Additive Control Group | 10 | 4 | 10.3 | 7.2 |
| A Additive Group (Comparative Group) | 10 | 1 | 11.3 | 10.5 |
| B Additive Group (Comparative Group) | 10 | 2 | 11.1 | 10.1 |
| C Additive Group (Group which received preparations of Invention) | 10 | 0 | 12.8 | 11.6 |

An asterisk (*) designates mean values based on breeding sows free from death abortions.

As is clear from the above test results, the numbers of death abortions in the Additive-free Control Group, B Additive Group (Comparative Group), A Additive Group (Comparative Group), and C Additive Group (Group to which preparations of the Present Invention is Added) were 4/10 (40.0%), 2/10 (20.0%), 1/10 (10.0%), 0/10 (0.00%) in descending order, respectively. That is, the Control Group had the largest number of death abortions. The number of born pigs was 10.3 in the Control Group, 11.1 in B Additive Group, 11.3 in A Additive Group and 12.8 in C Additive Group in ascending order, that is, B is 0.8, A is 1.0, C is 2.5 larger than Control Group in number. Also in the number of pigs at the time of weaning for the Control Group was 7.2, B was 10.1, A was 10.5, and C was 11.6, that is, Additive Groups B, A, and C were 2.9, 3.3, and 4.4 larger than Control Group in number, respectively. From these facts, it has been proved that the bacterial cell powder and their culture residue powder produced in Examples 1[b] and 1[c], respectively, are more effective at preventing piglet death due to abortion caused by hog herpesvirus type I and thus increase the survival of parturition pigs compared to the additive-free case. Especially, the mixture of Examples 1[b] and 1[c] showed further superior effects compared to single ingredient dosages.

## TEST EXAMPLE 3

Test for Escherichia coli, *Salmonella* Enteritidis, and *Salmonella* Typhimurium

Animals and Germs to be Tested

Animals to be tested were ICR family male mice (six weeks after birth), and groups each consisting of ten mice were used for each dosage and for the control. Challenge microorganisms were Escherichia coli (sepsis type), *Salmonella* Enteritidis (food poisoning origin strains), and *Salmonella* Typhimuriums (ATCC strain No. 1311).

## COMPARISON TEST 1

Five groups were dosed with 1000 μg, 500 μg, 100 μg, 50 μg, and 25 μg per mouse of lyophilized bacterial cells without capsules produced in Example 1 [b], and one non-dosed group (control group) were prepared. Dehydrated bacterial cells were administered into the abdominal cavity seven days and two days before the challenge by pathogens. The challenge microorganisms, Escherichia coli (2.1 x $10^6$ CFU (Colony Forming Unit) per mouse), *Salmonella* Enteritidis (2.4 x $10^7$ CFU per mouse), or *Salmonella* Typhimurium (2.9 x $10^7$ CFU per mouse) were inoculated into the abdominal cavity of the mouse. The effects were judged on the basis of survival ratio, which was given as survival ratio = (the number of surviving mice in a group dosed with dehydrated bacterial cells -the number of surviving mice in the control group) / the number of mice in the group x 100 where

◎ : ( = 75 %, extremely effective)
○ : (50 to 75 %, effective)
Δ : (25 to 50 %, somewhat effective)
X : ( = 25%, ineffective)

Table 4 shows the above test results.

[TABLE 4]

| Dosage per Mouse | Mice Survival Effects | | |
| --- | --- | --- | --- |
| | Escherichia coli | *Salmonella* Enteritidis | *Salmonella* Typhimurium |
| 1000 | ◎ | ◎ | ○ |
| 500 | ◎ | ◎ | ○ |
| 100 | ◎ | ◎ | ○ |
| 50 | ◎ | ◎ | X |
| 25 | ◎ | ○ | X |

As can be clearly seen from the above results, against Escherichia coli and *Salmonella* Enteritidis, each dosage group was "extremely effective" (excepting "effective" for 25 μg dosage group of Salmonella Enteritidis). Furthermore, against Salmonella Typhimurium (mouse died where < 10 CFU/mouse) dosage groups of 50 ug or less were "ineffective" while dosage groups of 100 ug or more were "effective".

Mice in the non-dosage control group all died one to five days after the challenge by each challenge microorganism .

From the above tests, it appears that,in the mouse, bacterial cells without capsules are effective against Escherichia coli and *Salmonella* Enteritidis, but ineffective against *Salmonella* Typhimurium with 50 ug or less per mouse dosage.

COMPARISON TEST 2

Test feeds including 0.2 wt%, 0.5 wt%, 0.75 wt%, and 1.0 wt% of bacteria culture residue powder produced in Example 1 [c] respectively as the additive, and additive-free feed (control) were fed to mice for three weeks, and thereafter challenge tests were carried out in the same manner as Comparison Test 1 by the use of Escherichia coli, *Salmonella* Enteritidis, and *Salmonella* Typhimurium. The symbols used represent the same values as in Comparison Test 1.

The above test results are shown in Table 5.

[TABLE 5]

| Test Groups | | Mice Survival Effects | | |
|---|---|---|---|---|
| | | Escherichia coli | *Salmonella* Enteritidis | *Salmonella* Typhimurium |
| Control Group | | D* | D | D |
| Additive Groups(%) | 0.2 | × | × | × |
| | 0.5 | ◎ | ○ | × |
| | 0.75 | ◎ | ○ | ○ |
| | 1.00 | ◎ | ○ | ○ |

An asterisk (*) designates that mice died  one to five days after the challenge. "D" indicates that the mice in the group all died following micro-organism challenge.

The above test results proved that against Escherichia coli 0.5 wt% or more dosage groups were "extremely effective", while against *Salmonella* Enteritidis 0.5 wt% or more dosage groups, and against *Salmonella* Typhimurium dosage groups with 0.75 wt% and 1.00 wt% were "effective".

TEST EXAMPLE OF THE INVENTION

Mice were fed for three weeks with four kinds of test feeds and non-additive feed (control), the test feeds containing as the additives 0.5 wt % of culture residue powder cleared of bacterial cells produced in Example 1 [c], and respectively 0.005 wt%, 0.01 wt%, 0.02 wt%, and 0.05 wt% of bacterial cells without capsules produced in Example 1 [b]. Then, using Escherichia coli, *Salmonella* Enteritidis, and *Salmonella* Typhimurium, the challenge tests were performed in the same manner as in Comparison Test 1. The symbols used have the same meaning as in Comparison Test 1.

The above test results are shown in Table 6.

[TABLE 6]

| Test Groups | Mice Survival Effects | | |
|---|---|---|---|
| | Escherichia coli | *Salmonella* Enteritidis | *Salmonella* Typhimurium |
| Control Groups | D *1 | D | D |
| Bacterial Cells (0.005) Culture Residue (0.5) | ◎ | ◎ | ✕ |
| Bacterial Cells (0.01) Culture Residue (0.5) | ◎ | ◎ | ◯ |
| Bacterial Cells (0.02) Culture Residue (0.5) | ◎ | ◎ | ◯ |
| Bacterial Cells (0.05) Culture Residue (0.5) | ◎ | ◎ | ◯ |

*1 indicates that the mice died in one to 5 days after the challenge. "D" indicates that all mice in the group died following micro-organism challenge.

The amounts of Bacterial Cell Powder and Culture Residue are all given as a wt % of the food given to the mice.

As is apparent from the above test results, against Escherichia coli and *Salmonella* Enteritidis, each of dosage, groups were "extremely effective", while against *Salmonella* Typhimurium dosage groups of 0.01 wt% or more were "effective".

The above test results have proved that the mixture of the bacterial culture residue and the bacterial cells without capsules (which are both active ingredients of the present invention) has a higher protective activity and anti-microorganism activity against high-pathogenic microorganisms such as Escherichia coli, *Salmonella* Enteritidis, and *Salmonella* Typhimurium, as compared with their respective single dosage.

The immunity activating function in a living body caused by the bacterial cells and their culture residue preparations of the present invention were subjected to tests.

TEST EXAMPLE 4

Immunity Activity Test

Mouse feed on the market was dosed with A (0.005 wt% of bacterial cell powder), B (0.5 wt% of culture residue powder), and C (0.005wt% of bacterial cell powder + 0.5 wt% of cultureesidue powder) respectively, to prepare additive containing feeds to test. In this test, the bacterial cell powder produced in Example 1 [b], and the culture residue powder produced in Example 1 [c] were used.

The above described test feeds including additives A, B, and C, and an additive-free feed (control) were each fed to groups of mice each consisting of five ICR family male mice for 3 weeks. After feeding test additive-loaded feeds, the lymph nodes of the spleen and the mesentery of the mouse to be tested were extracted to prepare lymphocytes. Concanavalin A (Con A) was added to thus prepared lymphocytes, which was cultured for 72 hours at 37°C under 5% $CO_2$. Then the obtained culture skim was subjected to lymphoblastogenesis (lymphocyte blastoid formation) Using the value of lymphocytes for the additive-free feed group as a control value, the lymphoblastogenesis ratio can be given by the following expression.

$$\text{Lymphoblastogenesis ratio} = \frac{\text{experiment measured value}}{\text{control value}} \times 100$$

Test results are shown in Table 7.

[TABLE 7]

| Origin | Test Groups (%) | | | |
|---|---|---|---|---|
| Internal | Additive- | Additive-loaded Groups | | |
| Organs | free Group | A | B | C |
| | (Control) | (Comparison) | (Comparison) | (preparations of Invention) |
| Spleen Lymph Node | 0 | 3 | 5 | 6 |
| Mesentery Lymph Node | 0 | 15 | 4 | 18 |

As is clearly seen from the above test results, lymphoblastogenesis ratio was nil in the control group, while it was rather high in all additive-loaded groups, markedly in group C. Furthermore, in groups A and C the lymphocyte blastoid formation rate was higher in effector cells originated from the mesentery lymph node than from the spleen, whereas regarding group B it was higher in effector cells originated from the spleen than from the mesentery lymph node. This proves that among the test additives the mixture additive is remarkably superior in activating non-specific cellular immunity.

Toxicity tests are described below.

TEST EXAMPLE 5

Male or female ICR family mice (6 weeks after birth, weight of 21 ± 2 g) were used as test animals. The bacterial cell powder obtained in Example 1 [b] suspended in sterilized physiological salt solution was administered into the abdominal cavity of the mouse in a dosage of 5 mg per 1 Kg mouse weight for A, 10 mg for B, and 100 mg for C; the culture residue powder obtained in Example 1 [c] in a dosage of 50mg/kg mouse weight for D, 500 mg per 1 Kg for E and 5000 mg for F; and the bacterial cell powder and the culture residue powder were given in the dosage of 5 mg and 50 mg respectively per 1 Kg mouse weight for G, and 10 mg and 500 mg for H, and 100 mg + 5000 mg for I, each for ten consecutive days. For the control group, 5 % of mannitol solution was administered for ten consecutive days. There are ten mice in each group.

One month after administration the number of dead mice in each group was counted.

The test results are shown in Table 8.

[TABLE 8]

| Test Groups | | Sex Distinction | Number of Test Mice | Number of Dead Mice | Mortality Rate (%) |
|---|---|---|---|---|---|
| Control Group | | m | 10 | 0 | 0 |
| | | f | 10 | 0 | 0 |
| Dosage Groups | A | m | 10 | 0 | 0 |
| | | f | 10 | 0 | 0 |
| | B | m | 10 | 0 | 0 |
| | | f | 10 | 0 | 0 |
| | C | m | 10 | 0 | 0 |
| | | f | 10 | 0 | 0 |
| | D | m | 10 | 0 | 0 |
| | | f | 10 | 0 | 0 |
| | E | m | 10 | 0 | 0 |
| | | f | 10 | 0 | 0 |
| | F | m | 10 | 0 | 0 |
| | | f | 10 | 0 | 0 |
| | G | m | 10 | 0 | 0 |
| | | f | 10 | 0 | 0 |
| | H | m | 10 | 0 | 0 |
| | | f | 10 | 0 | 0 |
| | I | m | 10 | 0 | 0 |
| | | f | 10 | 0 | 0 |

As is apparent from the above test results, no mice died in any group. Moreover, anaphylaxis (a type of acute toxicity which might appear after successive dosages of antigenic substances) was not observed either.

The preparations and food additives in accordance with the the present invention are remarkably effective against bacterial and viral infectious diseases, and are free from toxicity. They can be taken either in dosage form or together with food,to enhance the immune activity of humans, and in particular, of babies or infants, and to prevent infection by bacteria or viruses, particularly against herpesvirus or to alleviate clinical conditions. Thus, the present invention can be said to be extremely useful.

**Claims**

1. A composition comprising a bacterial culture residue concentrate and decapsulated bacterial cells.

2. A composition as claimed in claim 1 wherein the ratio of decapsulated bacterial cells : bacterial culture residue concentrate is in the range of 0.001 - 0.5 : 1.

3. A composition as claimed in either of claims 1 and 2 wherein the bacteria used to produce said bacterial culture residue concentrate are selected from Bifidobacterium, Bacillus, Lactobacillus and non-pathogenic strains of Streptococcus.

4. A composition as claimed in either of claims 1 and 2 wherein said bacteria used to produce said decapsulated bacterial cells are selected from Bifidobacterium, Bacillus, Lactobacillus and non-pathogenic strains of Streptococcus.

5. A composition as claimed in either of claims 3 and 4 wherein said bacteria are Bifidobacteria.

6. A composition as claimed in any one of claims 1 to 5 wherein said decapsulated bacterial cells are produced by enzymic or surfactant treatment or by stirring or shaking of capsulated bacteria.

7. A composition as claimed in any one of claims 1 to 6 wherein said bacterial culture residue concentrate is produced by culturing bacteria in a suitable medium, subsequent removal of said bacteria by dialysis or centrifugation to give a culture residue and then condensing said residue by 5 to 20 times.

8. A composition as claimed in any of claims 1 to 7 where said bacterial culture residue concentrate is dehydrated, optionally by spray or freeze drying.

9. The use of a composition as claimed in any one of claims 1 to 8 as a food additive or as an active ingredient in a pharmaceutical.